(19) Europäisches Patentamt
European Patent Office
Office européen des brevets

(11) Publication number: **0 066 208**
**B1**

(12) # EUROPEAN PATENT SPECIFICATION

(45) Date of publication of patent specification: **02.10.85**

(21) Application number: **82104433.6**

(22) Date of filing: **19.05.82**

(51) Int. Cl.⁴: **C 07 D 473/18,** A 61 K 31/52 // C07C43/174, C07C43/178, C07C69/18

(54) **9-(1,3-Dihydroxy-2-propoxymethyl)-guanine as antiviral agent.**

(30) Priority: **21.05.81 US 267210**

(43) Date of publication of application:
**08.12.82 Bulletin 82/49**

(45) Publication of the grant of the patent:
**02.10.85 Bulletin 85/40**

(84) Designated Contracting States:
**AT BE CH DE FR GB IT LI LU NL SE**

(56) References cited:
**EP-A-0 049 072**
**US-A-4 199 574**

**TETRAHEDRON LETTERS, vol. 21, 1980, OXFORD (GB), K.K. OGILVIE et al.: "Ring open analogues of deoxynucleotides" pages 327-330**

**Doctoral thesis M.F.GILLEN: "Synthesis and properties of novel nucleoside and nucleotide analogues", Montreal, April 1980**

(73) Proprietor: **SYNTEX (U.S.A.) INC.**
**3401 Hillview Avenue**
**Palo Alto, California 94304 (US)**

(72) Inventor: **Verheyden, Julien P.**
**244 Marvin Avenue**
**Los Altos California 94022 (US)**
Inventor: **Martin, John C.**
**3526 Page Street**
**Redwood City California 94063 (US)**

(74) Representative: **Schmied-Kowarzik, Volker, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

EP 0 066 208 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

*Field of the Invention*

This invention relates to 9-(1,3-dihydroxy-2-propoxymethyl)guanine and pharmaceutically acceptable salts thereof which are useful as antiviral agents. The invention also relates to a pharmaceutical composition containing the above compound in combination with a suitable non-toxic excipient, the composition being useful in combatting viral infections. The invention also relates to a process for preparing the compound of the invention.

*Related Disclosure*

Viral infections are widespread and result in a wide variety of symptoms. Some viral infections are easily overcome by the body's defense mechanism while others are of a more serious nature leading to permanent damage, e.g., blindness, and even to death. One such family of viruses which may cause serious infections is the Herpes virus group.

The drugs presently used to treat viral infections are ineffective in many cases or, if effective, are needed in large and/or continuous dosages which produce serious side-effects and/or toxicity. Therefore there is a need for an effective antiviral agent which is effective at lower dosages than the presently available drugs, thus diminishing the chance of possible side-effects and toxicity.

U.S. Patent No. 4,199,574 discloses compounds represented by the following generic formula:

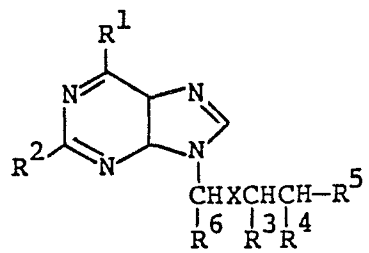

(A)

wherein X is sulphur or oxygen, $R^1$ is hydrogen, halogen, hydroxy, alkoxy, azide, thio, alkylthio, amino, alkylamino or dialkylamino; $R^2$ is hydrogen, halogen, alkylthio, acylamino, amino or azide; $R^3$ is hydrogen, straight or branch chain or cyclic alkyl, hydroxyalkyl, benzyloxyalkyl or phenyl; $R^4$ is hydrogen, hydroxy or alkyl; $R^5$ is hydrogen, hydroxy, amino, alkyl, hydroxyalkyl, benzyloxy, benzoyloxy, benzoyloxymethyl, sulphamoyloxy, phosphate, carboxypropiamyloxy, straight chain or cyclic acyloxy having from 1 to 8 carbon atoms e.g., acetoxy or substituted carbamoyl group of formula NHCO—Z wherein Z is alkyl, aryl or aralkyl optionally substituted by one or more of sulphonyl, amino, carbamoyl or halogen; $R^6$ is hydrogen or alkyl, provided that when X is oxygen and $R^2$, $R^3$, $R^4$, and $R^6$ are hydrogen, $R^1$ is not amino or methylamino when $R^5$ is hydrogen or hydroxy, or a salt thereof.

The class of compounds represented by the above formula and the pharmaceutically acceptable acid addition salts thereof are described to exhibit antiviral activity. See also *Tetrahedron Letters, 21,* 237—30 (1980). In EP - A - 49072 a series of antiviral pyrine and pyrimidine compounds are disclosed. This application was filed with the European Patent Office on 15.9.81.

Summary of the Invention

It has now been discovered that, surprisingly, thė compound, 9-(1,3-dihydroxy-2-propoxymethyl)-guanine and its salts is a particularly active antiviral agent. The selective activity of this compound is highlighted when the compound is compared with the structurally most similar compounds disclosed in U.S. 4,199,574 in an antiviral assay as shown in detail in Example 5.

The first aspect of the present invention is the compound 9-(1,3-dihydroxy-2-propoxymethyl)guanine which may be represented by the formula

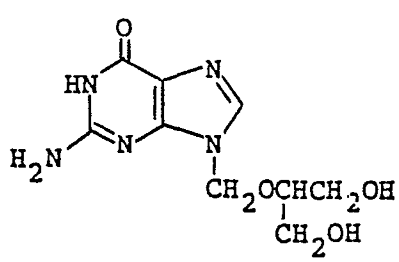

(I)

and the pharmaceutically acceptable salts thereof.

Another aspect of the invention relates to pharmaceutical compositions which contain, as the essential ingredient, the compound of the instant invention. These compositions may contain other pharmaceutically active ingredients of the same indication. In most cases these compositions will contain suitable pharmaceutical carriers or excipients.

Yet another aspect of the invention is a process for preparing the compound of formula (I) and its pharmaceutically acceptable salts thereof which comprises

a) deacylating a compound of the formula

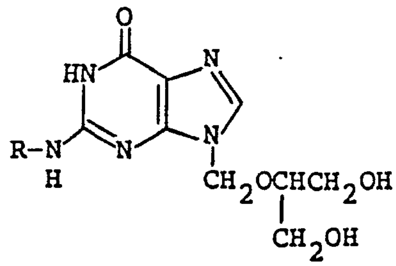

wherein R is defined as an acyl group,

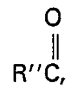

wherein R″ is a straight or branched hydrocarbon chain of one to ten carbon atoms to afford 9-(1,3-dihydroxy-2-propoxymethyl)guanine; or

b) dearylkylating a compound of the formula

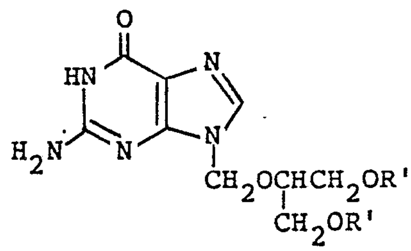

wherein R′ is defined as a protecting group such as benzyl optionally substituted with 1 or 2 alkoxy groups, where alkyl is as herein defined, preferably methoxy, or lower alkyl groups, where alkyl is defined as a straight or branched chain hydrocarbon of one to four carbon atoms, preferably methyl, to afford 9-(1,3-dihydroxy-2-propoxymethyl)guanine; or

c. dearylalkylating and deacylating a compound of the formula

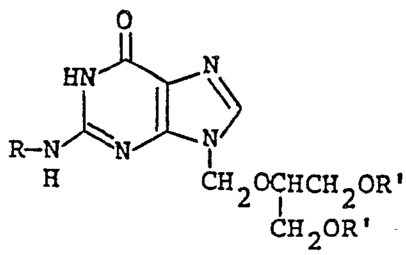

wherein R and R′ are as herein defined to afford 9-(1,3-dihydroxy-2-propoxymethyl)guanine; or

d) converting 9-(1,3-dihydroxy-2-propoxy-methyl)guanine to a pharmaceutically acceptable salt; or

e) converting a salt of 9-(1,3-dihydroxy-2-propoxymethyl)guanine to 9-(1,3-dihydroxy-2-propoxy)-guanine; or

f) converting a salt of 9-(1,3-dihydroxy-2-propoxymethyl)guanine to a pharmaceutically acceptable salt thereof.

Another aspect of the invention are the compounds of formula IX, wherein

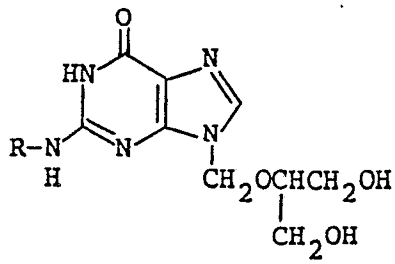

(IX)

and the salts thereof wherein R is an acyl group, R''CO, wherein R'' is a straight or branched hydrocarbon chain of one to ten carbon atoms which comprises

a) dearalkylating a compound of the formula

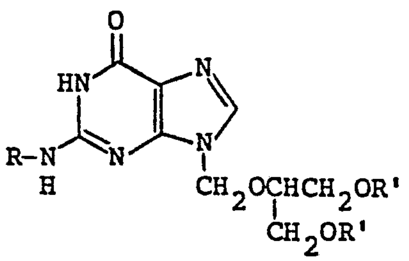

(VIII)

wherein R is as hereinabove defined and R' is a protecting group such as benzyl optionally substituted with 1 or 2 alkoxy groups, or lower alkyl groups wherein alkyl is defined as a straight or branched hydrocarbon chain of one to four carbon atoms; or

b) reacting a compound of formula (VIII) with a Lewis acid; or

c) reacting a compound of formula (VIII) with acetic acid; or

d) converting compound of formula (IX) to its salt; or

e) converting the salt to the corresponding free compound of formula (IX).

These compounds of formula IX wherein R is an acyl group as defined above serve as intermediates for the compound of formula I in which process these compounds are deacylated as described in detail below. Another aspect of the invention is a process for preparing the compounds of formula IX which is described in more detail below.

Detailed Description and Preferred Embodiment

As used in the specification and appended claims, unless specified to the contrary, the following terms have the meaning indicated.

"Pharmaceutically acceptable salts" refers to those salts which possess the biological effectiveness and properties of the free compound and which are not biologically or otherwise undesirable. The salts may be either the acid addition salts or inorganic metal salts, preferably alkaline earth metal salts. Suitable acids for salt formation are inorganic acids such as hydrochloric acid, hydrobromic acid, sulfuric acid, nitric acid, phosphoric acid and the like, and organic acids such as trifluoroacetic acid, menthanesulfonic acid, ethanesulfonic acid, p-toluenesulfonic acid and the like. Suitable bases for salt formation are organic bases, inorganic metal bases such as copper or silver bases, and alkali metal bases such as alkali metal hydroxides, e.g., sodium hydroxide, potassium hydroxide and the like.

The compound of the present invention characterized by formula (I) above, and the pharmaceutically acceptable salts thereof, are distinguished by the discovery that the 1,3-dihydroxy-2-propoxymethyl group substitution at the 9-position of the guanine nucleus provides, surprisingly, a highly active compound.

Utility and Administration

The subject compound of formula (I) and the pharmaceutically acceptable salts thereof exhibit potent antiviral activity when administered to animals, particularly mammals (non-human or human), cold-blooded animals such as fish, and birds, most particularly humans. For example, the compound of the present invention exhibits excellent activity against Herpes Simplex virus I and II and related viruses such as cytomegalovirus. Epstein-Barr virus and varicella Zoster virus.

Pharmaceutical compositions, both veterinary and human, containing the subject compound appropriate for antiviral use are prepared by methods, and may contain excipients which are well known in the art. A generally recognized compendium of such methods and ingredients is *Remington's Pharmaceutical Sciences* by E. W. Martin, (Mark Publ. Co., 15th Ed., 1975).

The pharmaceutical compositions of the invention may also contain other antivirally active substances. In general, the pharmaceutical composition may contain from 0.1 to 90% by weight of the active substance.

The compound of the invention may be administered parenterally (for example, by intravenous, intraperitoneal or intramuscular injection), orally, topically or rectally, depending on whether the preparation is used to treat internal or external viral infections.

For internal infections the compositions are administered orally or parenterally at dose levels, calculated as the free base, of about 0.1 to 300 mg/kg, preferably 1.0 to 30 mg/kg of mammal body weight, and may be used in man in a unit dosage form, administered one to four times daily in the amount of 1 to 250 mg per unit dose. For oral administration, fine powders or granules may contain diluting, dispersing and/or surface active agents, and may be presented in a draught, in water or in a syrup; in capsules or sachets in the dry state or in a non-aqueous solution or suspension, wherein suspending agents may be included; in tablets, wherein binders and lubricants may be included; or in a suspension in water or a

syrup. Where desirable or necessary, flavoring, preserving, suspending, thickening or emulsifying agents may be included. Tablets and granules are preferred, and these may be coated.

For parenteral administration or for administration as drops, as for eye infections, the compounds may be presented in aqueous solution in a concentration of from about 0.1 to 10%, more preferably about 0.1 to 7%. The solution may contain antioxidants, buffers, etc.

Alternatively for infections of the eye, or other external tissues, e.g. mouth and skin, the compositions are preferably applied to the infected part of the body of the patient topically as an ointment, cream, aerosol or powder, preferably as an ointment or cream. The compounds may be presented in an ointment, for instance with a water soluble ointment base, or in a cream, for instance with an oil in water cream base, in a concentration of from about 0.01 to 10%; preferably 0.1 to 7%, most preferably about 0.5% w/v. Additionally, viral infections of the eye, such as Herpetic keratitis may be treated by use of a sustained release drug delivery system as is described in U.S. 4,217,898.

The compositions of the present invention are also useful in treating non-human mammals, birds, e.g., chickens and turkeys, and cold-blooded animals such as fish. Avian viral diseases such as New Castle disease, Marek's disease and the like are prevented and/or treated by compounds of the present invention by methods well-known in the veterinary art such as by dipping the unhatched eggs into a solution containing the compound, injecting the birds with the composition, containing the compound, or by adding the compound of the instant invention to feed or drinking water.

Fish which are in a confined area such as a pool, aquarium or holding tank may also be treated for viral infections such as herpes like viruses, e.g., channel catfish virus (CCV), herpes-virus salomones, Nerka virus and the like by adding the compound directly to the water of the pool, aquarium or holding tank or by incorporating the compounds into the feed.

The exact regimen for administration of the compounds and compositions disclosed herein will necessarily be dependent upon the needs of the individual subject being treated, the type of treatment and, of course, the judgement of the attending practitioner.

Preparation

The compound of formula (I) may be prepared by the following reaction scheme wherein R is defined as an acyl group,

$$R''\overset{\overset{\displaystyle O}{\|}}{C},$$

wherein R'' is a straight or branched hydrocarbon chain of one to ten carbon atoms and R' is defined as a protecting group such as benzyl optionally substituted with 1 or 2 alkoxy groups, where alkyl is a straight or branched hydrocarbon chain of one to four carbon atoms, preferably methoxy, or lower alkyl groups, where alkyl is as herein defined, preferably methyl.

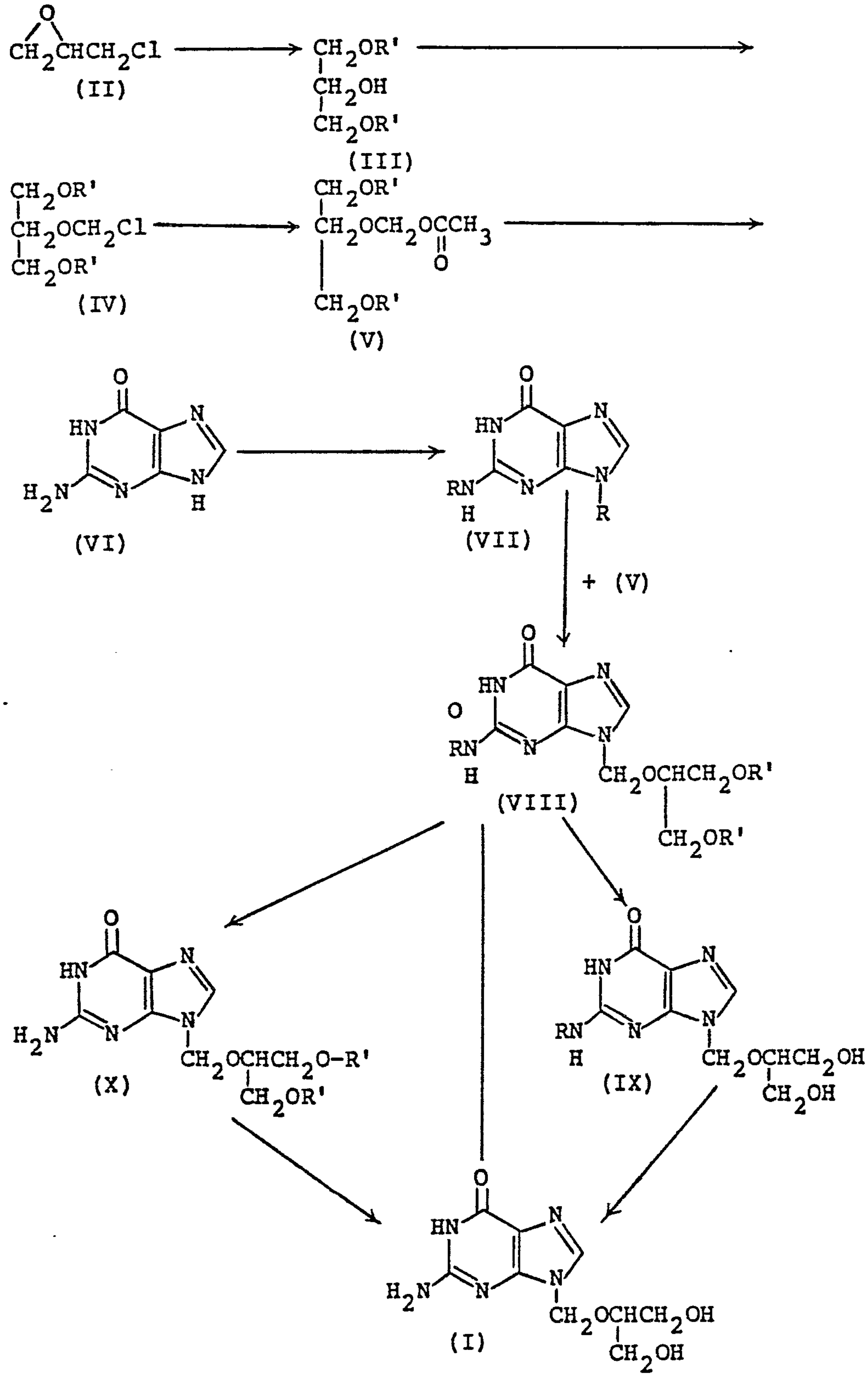


(II) (III) (IV) (V) (VI) (VII) (VIII) (X) (IX) (I)

The compound of formula (III) is prepared by adding epichlorohydrin (II) dropwise to a solution of an alkali metal salt, preferably the sodium salt, of benzyl alcohol optionally substituted as defined above in a solvent such as dimethylformamide, dimethylacetamide, hexamethylphosphoramide, dimethylsulfoxide, sulfolane, tetrahydrofuran, and dioxane at a temperature of about 0°C to 100°C, preferably at about 15°C to 40°C. The reaction mixture is stirred from about 10 hours to 24 hours, preferably from about 12 hours to 18 hours at a temperature of about 0°C to 100°C, preferably from about 20°C to 50°C.

Compound of formula (III) is chloromethylated to compound of formula (IV) by bubbling dry hydrogen

chloride gas in a solution of the compound and paraformaldehyde dissolved in a halogenated hydrocarbon solvent such as dichloroethane, chloroform, dichloromethane, and 1,1,2-trichloroethane cooled to a temperature of about 0°C to 25°C, preferably at a temperature of about 0°C. The hydrogen chloride gas is added over 30 minutes to 3 hours, preferably over 1 hour to 2 hours until the paraformaldehyde dissolves. The solution is held at a temperature from about 0°C to 10°C for about 12 hours to 48 hours, preferably from about 0° to 5°C for about 16 hours to 24 hours.

Compound of formula (V) is prepared by reacting an alkali metal acetate such as sodium acetate with compound of formula (IV) dissolved in a solvent such as dimethylformamide, tetrahydrofuran, dimethylacetamide, hexamethylphosphoramide, dimethylsulfoxide, sulfolane, and dioxane at a temperature of about 0°C to 45°C, preferably from about 0°C to 25°C. The solution is stirred from about 5 to about 24 hours, preferably from about 10 hours to about 18 hours at a temperature of about 10°C to about 30°C, preferably at a temperature of about 15°C to 25°C.

Compound of formula (VII) is prepared by heating guanine (VI) with a carboxylic acid anhydride with 2 to 20 carbon atoms such as acetic anhydride butyric anhydride, valeric anhydride, caprylic anhydride, preferably acetic anhydride, neat, at reflux for about 10 to 24 hours, preferably for about 12 to 18 hours.

$N^2$,9-Diacylguanine of formula (VII) is reacted with compound of formula (V) to form compound of formula (VIII) neat or in a solvent such as dioxane, sulfolane and the like in the presence of a catalytic amount of an acid such as bis(p-nitrophenyl)phosphate, toluene sulfonic acid, methylphosphonic acid or dichloroacetic acid, preferably bis(p-nitrophenyl)phosphate at a temperature of about 75°C to 200°C, preferably at about 110°C to 180°C. The reaction is generally carried out using 0.8 moles to 1.2 moles of compound of formula (V) to one mole of compound of formula (VII).

The protecting groups can be removed both at once, optionally substituted benzyl then acyl, or acyl then optionally substituted benzyl.

The benzyl protecting (R′) groups can be removed first from compound of formula (VIII) by one of several methods; catalytic hydrogenation reaction with Lewis acids or acetolysis to form compound of formula (IX). The catalytic hydrogenation is generally conducted in the presence of a hydrogenation catalyst such as palladium, platinum, rhodium or nickel on a carrier such as carbon and in the presence of an inert solvent for the compound of formula IX. Hydrogen is added to the solution at a pressure of 15 psi to 200 psi, preferably at a pressure of 30 psi to 80 psi. The reaction temperature usually is in the range of 0 to 50°C. Under preferred conditions, palladium on carbon in a slurry is added to a solution of compound of formula (VIII) dissolved in a solvent such as an aqueous lower alkyl alcohol wherein alkyl is defined as a straight or branched chain hydrocarbon of one to four carbon atoms, preferably aqueous methanol.

The compound of formula I can be prepared by deacylating compound IX by removing the protecting group R in a manner known per se. This removal may be effected under solvolytic conditions, whereby a suitable solvent restores the amino function in position 2 of the guanine ring system. Such solvents include water or lower alkanols with 1 to 4 carbon atoms. The solvolysis can be conducted under acidic or basic conditions. The reaction temperatures are usually selected in the range of 0 to 50°C, preferably 10 to 30°C. Most preferred is the range of 15 to 25°C. Basic solvolsis is preferred. Suitable bases include alkali hydroxides, alkali carbonates, or ammonia. Lower alkanols are the preferred solvents, in particular methanol. Under the preferred conditions a solution of compound of formula (IX) and the base is stirred for about 1 hour to 36 hours, preferably for about 10 hours to 24 hours at a temperature of about 10°C to 30°C, preferably at a temperature of about 15°C to 25°C.

The acyl protecting group can be removed first, according to the same conditions as described above.

The benzyl (R′) protecting groups can be removed in a manner known per se from compound of formula (X) by one of several means; catalytic hydrogenation, reaction with Lewis acids, or acetolysis, to form compound of formula (I). The catalytic hydrogenation is generally conducted in the presence of a hydrogenation catalyst such as palladium, platinum, rhodium or nickel on a carrier such as carbon and in the presence of an inert solvent for the compound of formula X. Hydrogen is added to the solution at a pressure of 15 psi to 200 psi, preferably at a pressure of 30 psi to 80 psi. The reaction temperature usually is in the range of 0 to 50°C. Under preferred conditions, palladium on carbon in a slurry is added to a solution of compound of formula (X) dissolved in a solvent such as an aqueous lower alkyl alochol wherein alkyl is defined as a straight or branched chain hydrocarbon of one to four carbon atoms, preferably aqueous methanol.

Both acyl and benzyl protecting groups can be removed from compound of formula (VIII) in one step to form compound of formula (I).

Compound of formula (VIII) is refluxed (at a temperature between −25° and −40°C) in a solution of sodium in liquid ammonia, wherein sodium is present in a small excess, to which solution a small amount of a proton source such as a lower alkyl alcohol, wherein alkyl is a straight or branched hydrocarbon chain of one to four carbon atoms, preferably ethanol or methanol, has been added. The ammonia is evaporated off and the residue triturated with ammonium chloride to form a residue which is compound of formula (I).

The pharmaceutically acceptable salts of compound I are prepared by reacting the compound of formula I with a suitable base or acid as defined above. This conversion may be conducted in a solvent whereby approximately stoichiometric equivalents of the base or acid are added to compound of formula I in a suitable inert solvent at a temperature between 0 and 50°C.

Similarly the compounds of formula I can be liberated from salts of the compounds of formula I by

adding a stoichiometric equivalent of base to an acid additional salt or acid to a salt of a compound of formula I with a base, in a solvent at a temperature between 0 and 50°C.

Likewise, a salt of compound of formula I can be converted to a pharmaceutically acceptable salt of formula I for example by conventional salt double decomposition, whereby the anion or cation of the starting salt is replaced with the pharmaceutically acceptable anion or cation. The salt double decomposition usually takes advantage of the fact that the lesser soluble salt is prepared from the more soluble salt at double decomposition temperatures of 0 to 50°C.

The following specific description is given to enable those skilled in the art to more clearly understand and practice the invention. It is considered as being illustrative and representative thereof.

Preparation I

*Preparation of 1,3-Di-O-benzylglycerol*

Sodium hydride (100g (50% dispersion in mineral oil), 2.08 mol) was washed twice with one liter of hexane then dried under nitrogen. Dry dimethylformamide (1.5 l) was added. Benzyl alcohol (400 ml) was then added at such a rate to keep the temperature below 50°C. The addition took 2 hours. Epichlorhydrin (92.5g, 1 mol) was then added dropwise over 0.5 hour with ice cooling in order to keep the temperature below 40°C. The solution was next stirred for 16 hours at 21°C then for 2.5 hours at 50°C. The dimethylformamide was then removed by evaporation at reduced pressure. The oily residue was dissolved in 2.5 liters diethyl ether. The organic solution was washed with 2 liters of water, 2 liters of 2% hydrochloric acid, 2 liters of 1% sodium bicarbonate, and one liter of brine, dried over sodium sulfate, and concentrated to a brown oil. Distillation gave 147.8 g of 1,3-di-O-benzylglycerol (bp 170—180°C/l torr).

Preparation II

*Preparation of 1,3-Di-O-benzyl-2-O-chloromethylglycerol*

Dry hydrogen chloride gas was bubbled for 1.5 hours into a solution of 1,3-di-O-benzylglycerol from Preparation I (15 g, 55 mmol) and paraformaldehyde (3,3 g, 110 mmol) in 175 ml of 1,2-dichloroethane at 0°C. The solution was then stored in a stoppered flask for 21 hours at 4°C. Next, the solution was dried over magnesium sulfate with warming to 21°C then filtered and concentrated to give 17.5 g of 1,3-di-O-benzyl-2-O-chloromethylglycerol.

Preparation III

*Preparation of 2-O-Acetoxymethyl-1,3-di-O-benzylglycerol*

To a solution of 1,3-di-O-benzyl-2-O-chloromethylglycerol from Preparation II (17.5 g, 55 mmol) in 400 ml of dimethylformamide at 0°C under a drying tube was added sodium acetate (6 g). The solution was then warmed to 21°C and magnetically stirred for 15 hours. The solvent was removed by evaporation at reduced pressure and the oily residue dissolved in 1 pound of diethylether. The ether solution was washed once with 750 ml of water, two times with 250 ml of water, and once with 250 ml of brine, dried over sodium sulfate and concentrated to give 19 g of 2-O-acetoxymethyl-1,3-di-O-benzylglycerol as an oil.

Preparation IV

*Preparation of $N^2$,9-Diacetylguanine*

Guanine (20 g, 0.132 mol) was combined with 300 ml of acetic anhydride and the mixture heated at reflux for 16 hours. The mixture was cooled and the excess acetic anhydride removed by evaporation at reduced pressure. The residue was recrystallized from dimethyl sulfoxide to give 25.6 g of $N^2$,9-diacetylguanine.

Example 1

A) *Preparation of $N^2$-Acetyl-9-(1,3-dibenzyloxy-2-propoxymethyl)guanine*

$N^2$,9-Diacetylguanine from Preparation IV (15.61 g, 66 mmol), 2-O-acetoxymethyl-1,3-di-O-benzylglycerol from Preparation III (19 g, 55 mmol), and bis(p-nitrophenyl)-phosphate (0.5g) were stirred together with 150 ml of diethylether. The solvent was removed by evaporation and the residue heated in a 175°C oil bath for 1.5 hours under a stream of nitrogen. Column chromatography eluting with 1:9 methanol/methylene chloride followed by recrystallization from ethyl acetate afforded 4.76 g of $N^2$-acetyl-9-(1,3-dibenzyloxy-2-propoxymethyl)guanine, mp 145—146°C.

B) *Preparation of $N^2$-Acetyl-9-(1,3-dihydroxy-2-propoxymethyl)guanine*

To a solution of $N^2$-acetyl-9-(1,3-dibenzyloxy-2-propoxymethyl)guanine (4.62 g, 9.67 mmol) in 150 ml of methanol plus 40 ml of water was added 20% palladium hydroxide on carbon as a slurry in 10 ml of water. The mixture was hydrogenated on a Parr hydrogenator at 60 psi of hydrogen for 38 hours then filtered through celite and concentrated to a white solid. Recrystallization from methanol/ethyl acetate gave 1.4 g of $N^2$-acetyl-9-(1,3-dihydroxy-2-propoxymethyl)guanine, mp 205—208°C.

The mother liquor was further reduced with 10% palladium on carbon (1 g) in 150 ml of methanol plus 50 ml of water at 60 psi for 47 hours. The total yield of $N^2$-acetyl-9-(1,3-dihydroxy-2-propoxymethyl)guanine was 2.11 g.

C) *Preparation of 9-(1,2-Dihydroxy-2-propoxymethyl)guanine*

$N^2$-Acetyl-9-(1,3-dihydroxy-2-propoxymethyl)guanine (721.9 mg, 2.4 mmol) was stirred with 50 ml of methanolic ammonia solution (methanol saturated with ammonia at 0°C) for 17 hours at 21°C. The solution was concentrated to a white solid and the residue recrystallized from methanol to give 582.3 mg of 9-(1,3-dihydroxy-2-propoxymethyl)guanine, mp 250°C d.

Example 2

9-(1,3-Dihydroxy-2-propoxymethyl)guanine was dissolved in a solution of water containing one mole equivalent of sodium hydroxide. The solution was then lyophilized to give 9-(1,3-dihydroxy-2-propoxymethyl)guanine sodium salt as a white powder.

Example 3

The sodium salt of 9-(1,3-dihydroxy-2-propoxymethyl)-guanine was dissolved in a minimum amount of water and dilute hydrochloric acid was added to adjust the pH to 7. 9-(1,3-Dihydroxy-2-propoxymethyl)guanine, m.p. 250°C d crystallized from the solution.

Example 4

The following example illustrates the preparation of representative pharmaceutical formulations containing an active compound of Formula (I).

A. Topical Formulation

| | |
|---|---|
| Active compound | 0.2—2 g |
| Span 60 | 2 g |
| Tween 60 | 2 g |
| Mineral oil | 5 g |
| Petrolatum | 10 g |
| Methyl paraben | 0.05 g |
| Propyl paraben | 0.05 g |
| BHA (butylated hydroxy anisole) | 0.01 g |
| Water qs | 100 ml |

All of the above ingredients, except water, are combined and heated at 60°C with stirring. A sufficient quantity of water at 60°C is then added with vigorous stirring to provide 100 g of the cream formulation which is then cooled to room temperature.

The following formulation is useful for intraperitoneal and intramuscular injection.

B. IP and IM Formulation

| | |
|---|---|
| Active compound | 0.5 g |
| Propylene glycol | 20 g |
| Polyethylene glycol | 20 g |
| Tween 80 | 1 g |
| 0.9% Saline solution qs | 100 ml |

The active compound is dissolved in propylene glycol, polyethylene glycol 400 and Tween 80. A sufficient quantity of 0.9% saline solution is then added with stirring to provide 100 ml of the I.P. or I.M. solution which is filtered through a 0.2 micron membrane filter and packaged under sterile conditions.

The following formulation is useful for intravenous injection.

C. I.V. Formulation

| | |
|---|---|
| Active compound | 0.5 g |
| 0.9% Saline solution | 100 g |

The active compound is added to 100 ml of 0.9% saline solution with stirring to provide 100 ml of I.V. solution which is filtered through a 0.2 micron membrane filter and packaged under sterile conditions.

D. Tablet Formulation

| | Parts by weight |
|---|---|
| Active compound | 200 |
| Magnesium stearate | 3 |
| Starch | 30 |
| Lactose | 116 |
| PVP (polyvinylpyrrolidone) | 3 |

The above ingredients are combined and granulated using methanol as the solvent. The formulation is then dried and formed into tablets (containing 200 mg of active compound) with an appropriate tabletting machine.

The exceptional antiviral activity of the compound of the invention is illustrated by the following assay procedures:

The Herpes simplex virus 2 strain G for infection was prepared in HEp-2 cell cultures. Virus was adsorbed for 1 hour, fresh media was placed on the cells, and they were incubated at 35°C until all cells were infected. The cell suspension was frozen at −70°C, thawed, and centrifuged to remove cell debris. The supernatant fluid was aliquoted and stored frozen at −70°C until use. A $10^{6.7}$ dilution of the supernatant fluid produced a 50% cell culture infective dose ($CCID_{50}$) in HEp—2 cells and a $10^{3.7}$ dilution produced a 50% lethal challenge ($LC_{50}$) in mice.

Groups of 20 Swiss Webster female mice (15—17 gm), were challenged by intraperitoneal route using 0.2 ml of EMEM containing 10 $LC_{50}$/mouse of virus. Mice challenged with $10^{0.5}$ more or less virus than the 10 $LD_{50}$ challenge working properly.

Treatment with test compounds began 6 hours post-challenge. The mice, divided into groups of 20, were administered the compounds in saline s.c. at 5 mg/kg, 10 mg/kg and 20 mg/kg. One group of 20 mice was used as a control group and administered saline s.c. The treatment was repeated at 24, 48, 72 and 96 hours post-challenge.

Test compounds were the compound of the invention, and three compounds disclosed in U.S. 4,199,574 having structural similarity to portions of the compound of the invention.

| | | Survivers (out of 20) Days Post Challenge | | |
|---|---|---|---|---|
| Test Compound | Dosage | 12 | 14 | 21 |
| Untreated Controls | | 0 | 0 | 0 |
| 9-(1,3-dibenzoxy-2-propoxy methyl)adenine | 5 mg/kg | 2 | 2 | 2 |
| | 10 mg/kg | 1 | 0 | 0 |
| | 20 mg/kg | 1 | 1 | 1 |
| 9-(1,3-dibenzoxy-2-propoxy methyl)-6-mercaptopurine | 5 mg/kg | 2 | 0 | 0 |
| | 10 mg/kg | 0 | 0 | 0 |
| | 20 mg/kg | 1 | 0 | 0 |
| 9-(1-hydroxy-2-ethoxy methyl)guanine | 5 mg/kg | 1 | 1 | 1 |
| | 10 mg/kg | 3 | 3 | 3 |
| | 20 mg/kg | 5 | 4 | 4 |
| 9-(1,3-dihydroxy-2-propoxy methyl)guanine | 5 mg/kg | 15 | 14 | 14 |
| | 10 mg/kg | 19 | 18 | 18 |
| | 20 mg/kg | 19 | 18 | 15 |

In the above results, a comparison between the number of surviving animals administered the compound of the invention and the number of surviving animals administered the three prior art compounds clearly demonstrates the markedly superior antiviral activity of our compound.

**Claims**

1. The compound 9-(1,3-dihydroxy-2-propoxymethyl)guanine and the pharmaceutically acceptable salts thereof.

2. The compound 9-(1,3-dihydroxy-2-propoxymethyl)guanine.

3. The sodium salt of 9-(1,3-dihydroxy-2-propoxymethyl)guanine.

4. A pharmaceutical composition which comprises an effective amount of 9-(1,3-dihydroxy-2-propoxymethyl)guanine or a pharmaceutically acceptable salt thereof.

5. A process for preparing a 9-(1,3-dihydroxy-2-propoxy-methyl)guanine and the pharmaceutically acceptable salts thereof which comprises:

a) deacylating a compound of the formula

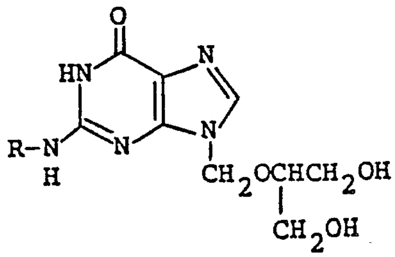

(IX)

wherein R is defined as an acyl group,

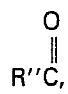

wherein R'' is a straight or branched hydrocarbon chain of one to ten carbon atoms, to afford 9-(1,3-dihydroxy-2-propoxymethyl)guanine; or

b) simultaneously dearylkylating and deacylating a compound of the formula

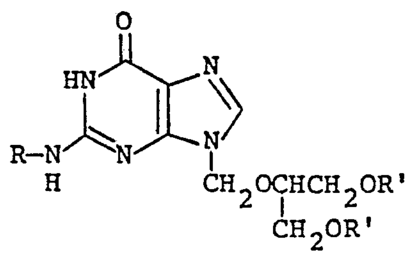

wherein R is as hereinabove defined and R' is a protecting group such as benzyl optionally substituted with 1 or 2 alkoxy groups, where alkyl is as herein defined, preferably methoxy, or lower alkyl groups, where alkyl is defined as a straight or branched chain hydrocarbon of one to four carbon atoms, preferably methyl, to afford 9-(1,3-dihydroxy-2-propoxymethyl)guanine; or

c) converting 9-(1,3-dihydroxy-2-propoxymethyl)guanine to a pharmaceutically acceptable salt; or

d) converting a salt of 9-(1,3-dihydroxy-2-propoxymethyl)guanine to 9-(1,3-dihydroxy-2-propoxymethyl)guanine; or

e) converting a salt of 9-(1,3-dihydroxy-2-propoxymethyl)guanine to a pharmaceutically acceptable salt thereof.

4. The compound of the formula

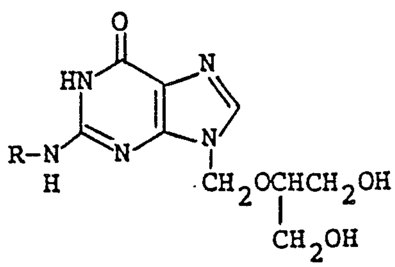

(IX)

and the salts thereof wherein R is an acyl group, R''CO, wherein R'' is a straight or branched hydrocarbon chain of one to ten carbon atoms.

7. The process for preparing a compound of the formula

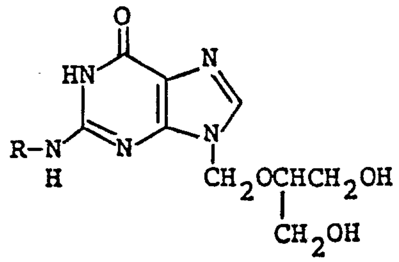

(IX)

and the salts thereof wherein R is an acyl group,

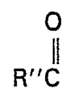

wherein R'' is a straight or branched hydrocarbon chain of one to ten carbon atoms which comprises

a) dearalkylating a compound of the formula

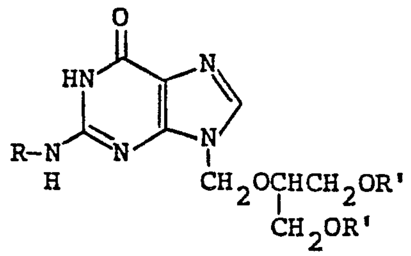

(VIII)

wherein R is as hereinabove defined and R' is a protecting group such as benzyl optionally substituted with 1 or 2 alkoxy groups, or lower alkyl groups wherein alkyl is defined as a straight or branched hydrocarbon chain of one to four carbon atoms; or

b) reacting a compound of formula (VIII) with a Lewis acid; or

c) reacting a compound of formula (VIII) with acetic acid; or

d) converting a compound of formula (IX) to its salt; or

e) converting the salt to the corresponding free compound of formula (IX).

**Patentansprüche**

1. Die Verbindung 9-(1,3-Dihydroxy-2-propoxymethyl)-guanin und die pharmazeutisch annehmbaren Salze davon.

2. Die Verbindung 9-(1,3-Dihydroxy-2-propoxymethyl)-guanin.

3. Das Natriumsalz von 9-(1,3-Dihydroxy-2-propoxymethyl)-guanin.

4. Eine pharmazeutische Zusammensetzung welche eine wirksame Menge von 9-(1,3-Dihydroxy-2-propoxymethyl)-guanin oder einem pharmazeutisch annehmbaren Salt davon unfaßt.

5. Ein Verfahren zur Herstellung von 9-(1,3-Dihydroxy-2-propoxymethyl)-guanin und den pharmazeutisch annehmbren Salzen davon, welches umfaßt.

a) die Deacylierung einer Verbindung der Formel

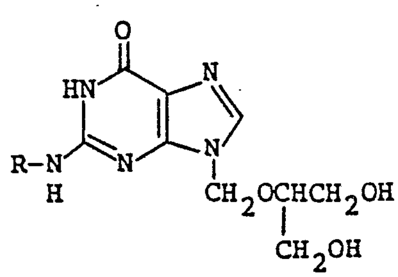

(IX)

worin R als eine Acylgruppe

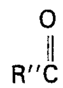

definiert ist, wobei R'' eine gerade oder verzweigte Kohlenwasserstoffkette mit 1 bis 10 Kohlenstoffatomen

ist, zu 9-(1,3-Dihydroxy-2-propoxymethyl)-guanin; oder die gleichzeitige Dearylalkylierung und Deacylierung einer Verbindung der Formel

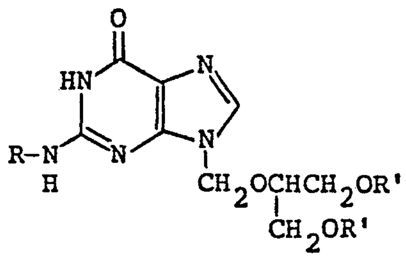

worin R wie oben definiert ist und R′ eine Schutzgruppe ist, z.B. Benzyl, das gegebenenfalls mit 1 oder 2 Alkoxygruppen, wobei Alkyl wie hierin definiert ist, vorzugsweise Methoxy, oder nierderen Alkylgruppen, wobei Alkyl als gerad- oder verzweigtkettiger Kohlenwasserstoff mit 1 bis 4 Kohlenstoffatomen definiert ist, vorzugsweise Methyl, substituiert ist, zu 9-(1,3-Dihydroxy-2-propoxymethyl)-guanin; oder

c) Überführen von 9-(1,3-Dihydroxy-2-propoxymethyl)-guanin in ein pharmazeutisch annehmbares Salz; oder

d) Überführen eines Salzes von 9-(1,3-Dihydroxy-2-propoxymethyl)-guanin in 9-(1,3-Dihydroxy-2-propoxy-methyl)-guanin; oder

e) Überführen eines Salzes von 9-(1,3-Dihydroxy-2-propoxymethyl)-guanin in ein pharmazeutisch annehmbares Salz davon.

6. Die Verbindung der Formel

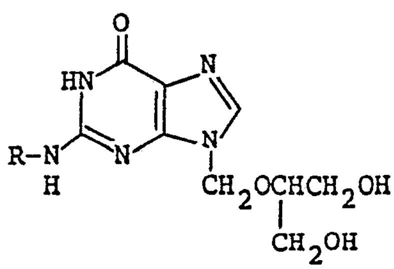

(IX)

und die Salze davon, worin R eine Acylgruppe R″CO ist, worin R″ eine gerade oder verzweigte Kohlenwasserstoffkette mit 1 bis 10 Kohlenstoffatomen ist.

7. Das Verfahren zur Herstellung einer Verbindung der Formel

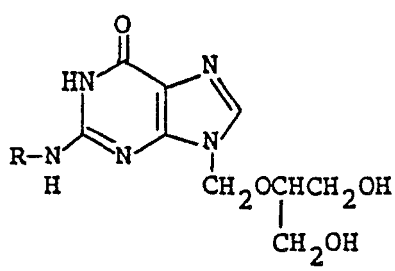

(IX)

und der Salze davon, worin R eine Acylgruppe

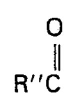

ist, worin R″ eine gerade oder vertweigte Kohlenwasserstoffkette mit 1 bis 10 Kohlenstoffatomen ist, welches umfaßt.

a) das Dearalkylieren einer Verbindung der Formel

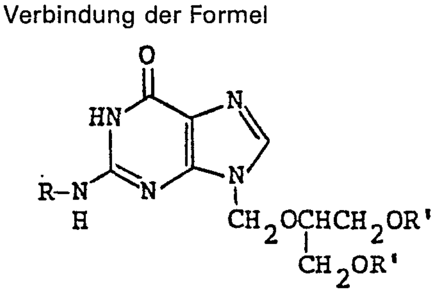

(VIII)

worin R wie oben definiert ist und R' eine Schutzgruppe ist, z.B. Benzyl, das gegenbenenfalls mit 1 oder 2 Alkoxygruppen oder niederen Alkylgruppen, worin Alkyl definiert ist als gerade oder verzweigte Kohlenwasserstoffkette mit 1 bis 4 Kohlenstoffatomen, substituiert ist; oder

b) Umsetzen einer Verbindung der Formel (VIII) mit einer Lewis-Säure; oder

c) Umsetzen einer Verbindung der Formel (VIII) mit Essigsäure; oder

d) Überführen einer Verbindung der Formel (IX) in ihr Salz; oder

e) Überführen des Salzes in die entsprechende freie Verbindung der Formel (IX).

**Revendications**

1. La 9-(1,3-dihydroxy-2-propoxyméthyl)guanine et ses sels pharmaceutiquement acceptables.

2. La 9-(1,3-dihydroxy-2-propoxyméthyl)guanine.

3. Le sel de sodium de la 9-(1,3-dihydroxy-2-propoxyméthyl)guanine.

4. Composition pharmaceutique, qui comprend une quantité efficace de 9-(1,3-dihydroxy-2-propoxyméthyl)guanine ou d'un pharmaceutiquement acceptable de ce composé.

5. Procédé de préparation de la 9-(1,3-dihydroxy-2-propoxyméthyl)guanine et de ses sels pharmaceutiquement acceptables, qui comprend:

a) la déacylation d'un composé de formule

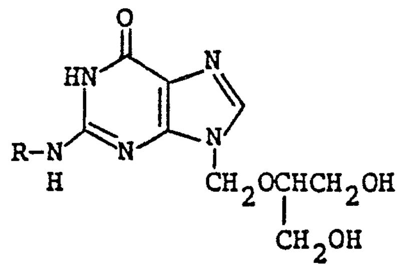

(IX)

dans laquelle R est défini comme un groupe acyle,

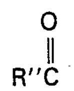

dans lequel R'' est une chaîne hydrocarbonée droite ou ramifiée ayant 1 à 10 atomes de carbone, pour former la 9-(1,3-dihydroxy-2-propoxyméthyl)guanine; ou

b) la déarylalkylation et las déacylation simultanées d'un composé de formule

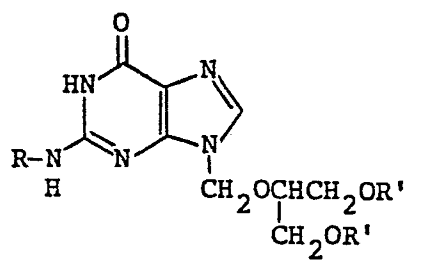

dans laquelle R a la définition donnée ci-dessus et R' est un groupe protecteur tel qu'un groupe benzyle portant éventuellement 1 ou 2 substituants alkoxy dont le radical alkyle est tel que défini ici, de préférence méthoxy, ou avec des groupes alkyle inférieurs, le groupe droite ou ramifiée ayant 1 à 4 atomes de carbone, de préférence le groupe méthyle, pour obtenir la 9-(1,3-dihydroxy-2-propoxyméthyl)guanine; ou

c) la transformation de la 9-(1,3-dihydroxy-2-propoxyméthyl)guanine en un sel pharmaceutiquement acceptable; ou

d) la transformation d'un sel de 9-(1,3-dihydroxy-2-propoxyméthyl)guanine en 9-(1,3-dihydroxy-2-propoxyméthyl)guanine; ou

e) la transformation de la 9-(1,3-dihydroxy-2-propoxyméthyl)guanine en un sel pharmaceutiquement acceptable.

6. Composé de formule

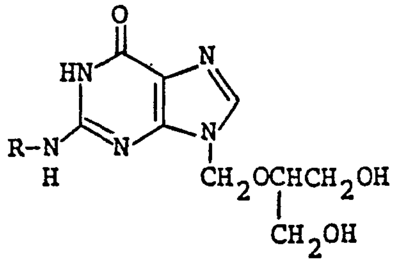

(IX)

et ses sels, formule dans laquelle R est un groupe acyle R''CO ou R'' est une chaîne hydrocarbonée droite ou ramifiée ayant 1 àyant 10 atomes de carbone.

7. Procédé de préparation d'un composé de formule

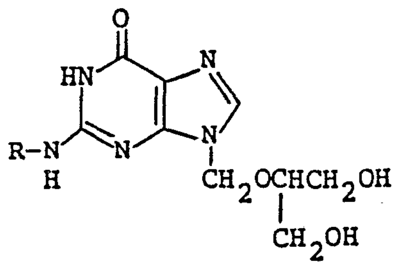

(IX)

et de ses sels, formule dans laquelle R est un groupe acyle

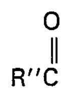

dans lequel R'' est une chaîne hydrocarbonée droite ou ramifiée ayant 1 à 10 atomes de carbone, qui comprend:

a) la déaralkylation d'un composé de formule

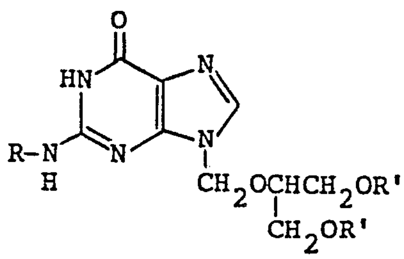

(VIII)

dans laquelle R a la définition donnée ci-dessus et R' est un groupe protecteur tel qu'un groupe benzyle éventuellement substitué avec un ou deux groupes alkoxy ou groupes alkyle inférieurs dont le radical alkyle est défini comme étant une chaîne hydrocarbonée droite ou ramifiée ayant 1 à 4 atomes de carbone; ou

b) la réaction d'un composé de formule (VIII) avec un acide de Lewis; ou

c) la réaction d'un composé de formule (VIII) avec l'acide acétique; ou

d) la transformation d'un composé de formule (IX) en son sel; ou

e) la transformation du sel en le composé libre correspondant de formule (IX).